# EUROPÄISCHE PATENTSCHRIFT

(11) **EP 0 360 118 B1**
(45) Veröffentlichungstag und Bekanntmachung des Hinweises auf die Patenterteilung: **12.05.1993**
(21) Anmeldenummer: 89116773.6
(22) Anmeldetag: 11.09.1989
(51) Int. Cl.: A61L 2/18, A01N 35/02, A01N 59/00, A61C 9/00

(54) **Verfahren zur Desinfektion von medizinischen Abformmassen**
Method of disinfecting medical impressions
Procédé de désinfection d'empreintes médicales

(30) Priorität: 19.09.1988 DE 3831779; 27.12.1988 DE 3844024
(43) Veröffentlichungstag der Anmeldung: 28.03.1990
(73) Patentinhaber: Henkel Kommanditgesellschaft auf Aktien, 40191 Düsseldorf (DE); ESPE Stiftung & Co Produktions- und Vertriebs KG, D-82229 Seefeld (DE)
(72) Erfinder: Hachmann, Klaus, Dr., D-4010 Hilden (DE); Bansemir, Klaus, Dr., D-4018 Langenfeld (DE); Disch, Karlheinz, Dr., D-5657 Haan (DE); Dasch, Walter, Dr., D-8000 München (DE); Pichl, Reinhard, Dr., D-8031 Hechendorf (DE); Ellrich, Klaus, Dr., D-8031 Wörthsee (DE)

(56) Entgegenhaltungen:
- EP-A- 0 016 319
- EP-A- 0 308 786
- WO-A-84/01894
- FR-A- 2 307 548
- GB-A- 2 090 140
- US-A- 3 912 450
- US-A- 3 983 252
- CHEMICAL ABSTRACTS, Band 109, Teil 8, 22. August 1988, Seite 435, Zusammenfassung Nr. 61410z, Columbus, Ohio, US; J. VIOHL et al.: "Dimensional stability of alginate impressions. Dimensional stability of alginate impressions and hardness of the gypsum casts during disinfection"

## Beschreibung

Auf vielen Gebieten der Medizin besteht, mehr oder weniger häufig, die Notwendigkeit, dimensionsgetreue Modelle von Organen oder Organteilen anzufertigen. So werden in der Pathologie und in der Chirurgie Modelle zu Demonstrations- oder Dokumentationszwecken benötigt; in der Orthopädie werden Modelle zur Anpassung von Prothesen verwendet, und in der Zahnmedizin wird anhand von Modellen Zahnersatz angefertigt. Zur Gewinnung des Modells wird dabei zunächst mit Hilfe von Abformmassen ein räumliches Negativ des betreffenden Organs hergestellt, das dann, gegebenenfalls nach Zwischenlagerung, als Form für die Anfertigung des eigentlichen Modells dient. Zur Herstellung des Negativs sind eine Reihe von Materialien entwickelt worden, die alle die Eigenschaft haben, zunächst plastisch verformbar zu sein und sich nach kurzer Zeit zu einer mehr oder weniger elastischen Masse zu verfestigen. Die Abformung geschieht in der Weise, daß man das betreffende Organ in das plastische Abformmaterial abdrückt und darin bis zur Verfestigung der Masse beläßt. Das Modell selbst wird dann meist im Gußverfahren hergestellt, indem beispielsweise Gemische aus Wasser und Gips in die Negativform eingetragen werden.

In der Zahnmedizin unterscheidet man zwischen starren und elastischen Abformmaterialien. Während starre Abformmaterialien wie Gips, Zinkoxid-Eugenolpasten, Wachse und Guttapercha hauptsächlich dafür eingesetzt werden, die Stellung der Zähne zueinander zu bestimmen, also die Bißregistrierung vorzunehmen, werden für die Herstellung von zahnärztlichen Modellen hauptsächlich elastische Abformmaterialien auf der Basis von synthetischen oder natürlichen Polymeren eingesetzt, bei denen die Verfestigung durch physikalische oder chemische Vernetzungsreaktionen zustande kommt.

Die heute wichtigsten synthetischen Polymere für diesen Zweck sind Silikone, Polyether und Polysulfide, die alle auf chemischem Wege vernetzt werden. Bei den Silikonmaterialien unterscheidet man prinzipiell zwischen den kondensationsvernetzenden Silikonmassen (die Härtung geschieht hierbei mit organischen Zinn- oder Titanverbindungen als Katalysatoren, die die Vernetzung der Ausgangspolysiloxane nach Abspaltungen von Endgruppen bewirken), zum anderen werden sogenannte additionsvernetzende Silikonabformmassen verwendet, bei denen die Härtung durch Reaktion eines Polysiloxans mit Vinyl-Endgruppen mit einem Polysiloxan mit SiH-Gruppen mittels bestimmter Platinkatalysatoren erfolgt. Neben den Polysulfiden spielen ferner die Polyethermaterialien aufgrund ihrer hydrophilen Materialeigenschaften eine große Rolle, da durch die hierbei mögliche gute Benetzung auch im wäßrigen Milieu die Zahnsituation im Mund besonders gut wiedergegeben werden kann. Die Vernetzungsreaktion hierbei beruht auf der Polymerisation von Epimin-endständigen Polyetherprepolymeren, welche durch Sulfoniumsalzkatalysatoren eingeleitet wird.

Die wichtigsten natürlichen Polymere sind polymere Kohlenhydrate, von denen wiederum die Alginate die höchste Bedeutung besitzen. Bei diesen Materialien bilden sich in der Erstarrungsreaktion wasserhaltige elastische Gele, deren Haltbarkeit geringer ist als die der synthetischen Massen.

Alginatabformmaterialien können bekanntermaßen Kieferabdrücke mit hinreichender Genauigkeit liefern und erfreuen sich aufgrund ihrer billigen Herstellbarkeit einer weiten Verbreitung. Geliefert wird dieses Abformmaterial dem Anwender in Form eines alginathaltigen Pulvers, welches von ihm mit einer definierten Menge Wasser vor der Anwendung vermischt wird. Hierbei gehen dann die Reaktanten Alginsäuresalz und Calciumsalz in Lösung, reagieren miteinander und bilden ein unlösliches, elastisches Hydrogel. Neben einem löslichen Alginsäuresalz wie Kalium- oder Natriumalginat und einem mäßig löslichen Calciumsalz wie Calciumsulfat, werden in der Regel Verzögerer wie Natriumphosphat oder Natriumpyrophosphat, Füllstoffe wie Kieselgur und komplexe Übergangsmetallfluoride wie Kaliumhexafluorotitanat eingesetzt. Vorteilhaft ist auch der Einsatz von geringen Mengen an pyrogener Kieselsäure zur Einstellung des thioxotropen Verhaltens. Zur Verhinderung der Staubwirkung dieser Alginatpulvermischung ist es bekannt, diese durch Zusatz von löslichen und unlöslichen organischen Verbindungen zu granulieren (z. B. EP-A 0 058 203 sowie DE-A 34 39 884 und DE-A 34 10 923).

Die Zusammensetzungen der Alginatabformmaterialien können in relativ weiten Bereichen variieren. So können 10 - 30 Gew.-% Natrium- oder Kaliumalginat, 10 - 30 Gew.-% Calciumsulfat, 0,5 - 5 Gew.-% Natriumphosphat und/oder Natriumpyrophosphat sowie als Rest übliche Füllstoffe, Aromastoffe sowie Thixotropie-Hilfsmittel enthalten sein. Als Füllstoffe sind besonders geeignet Kieselgure, Natrium- oder Kaliumhexafluorotitanat, sowie Zinkoxide.

Im Unterschied zu den Alginaten sind Abformmassen auf Agar-Agar-Basis reversible Materialien (sogenannte Hydrokolloide), die im Prinzip immer wieder verwendet werden können. Zur Abformung wird das stark wasserhaltige Agar-Agar-Gel in einem Wasserbad in den Solzustand überführt, das Abformmaterial in einem Löffel in den Mund gebracht und durch Zuführung von kaltem Wasser durch den Löffel in einen fest elastischen Zustand (Gel) zurückverwandelt.

In der Praxis werden die Negativform und das eigentliche Modell im allgemeinen nicht von derselben Person hergestellt. Vielmehr wird die Negativform nach der Abnahme vom Original über mehr oder weniger lange Transportwege an eine Modellwerkstatt übergeben, wo dann das Modell von eigens dafür geschultem Personal angefertigt wird. Bedingt durch den Herstellprozeß kann die Negativform in störender Weise mit Keimen belastet sein, wobei pathogene Keime nicht auszuschließen sind. Eine besondere Gefahr stellt dabei in der Zahnmedizin die Kontamination mit Hepatitis-Viren dar. Es ist deshalb schon sehr früh vorgeschlagen worden, die Negativform vor der Weitergabe zu desinfizieren, um eine Gefährdung der Personen, die später damit in Berührung kommen, auszuschließen.

Es gelang jedoch nicht, die für die Anwendung auf der Fläche und an medizinischen Geräten bekannten Desinfektionsmittel in zufriedenstellender Weise auch für Abformmassen zu verwenden. Die Schwierigkeiten beruhten darauf, daß ein meist anderes Keimspektrum auf einer völlig anderen Oberfläche zu bekämpfen war, die Abdruckmassen in ihrer Maßhaltigkeit und ihrer Oberflächengüte nicht durch den Desinfektionsprozeß beeinträchtigt werden durften und die Ausformbarkeit des eigentlichen Modells gewährleistet bleiben mußte. Insbesondere bei den hydrophilen Materialien wie Polyethern, Alginaten, Hydrokolloiden und hydrophilisierten Silikonen waren bei der Einwirkung von wäßrigen Desinfektionsmitteln aber auch von alkoholischen Desinfektionsmitteln auf die ausgeformte Masse starke Einflüsse auf die Qualität des danach hergestellten Modells zu beobachten. Um diese Schwierigkeiten zu umgehen, ist als Alternative beispielsweise vorgeschlagen worden, dem Abformmaterial schon vor der Abformung einen antimikrobiell wirkenden Stoff zuzusetzen, so daß nachträgliche Einwirkungen auf die Form unterbleiben können (G. Lott, H. K. Gribi, Quintessence international, Vol. 19, No. 8, 571 (1988); s. a. EP 265 776 und DD 244 068). Auch dieses Verfahren löst aber nicht alle Probleme, da viele an sich gut wirksame Desinfektionsmittel bei dieser Anwendung nicht ihre volle Wirksamkeit entfalten oder aber mit dem Abformmaterial oder der Mundschleimhaut unverträglich sind. Weiterhin wird eine Kontamination des Löffels, in dem sich die Form befindet, nicht beseitigt. Desinfektionsmittel für medizinische Instrumente und Wände, die mittlere Mengen an Alkoholen enthielten, wie beispielsweise in US 3 912 450 und FR 2 307 548 beschrieben, waren bisher für die Verwendung an Abformmassen nicht vorgeschlagen worden.

Der vorliegenden Erfindung lag in diesem Zusammenhang die Aufgabe zugrunde, ein für die nachträgliche Desinfektion der ausgeformten Masse geeignetes Verfahren zu finden. Diese Aufgabe konnte gelöst werden durch die Anwendung von Desinfektionsmitteln in verdünnter wäßrig alkoholischer Lösung.

Gegenstand der Erfindung ist daher ein Verfahren zur Desinfektion medizinischer Abformmassen, bei dem die Abformmassen nach der Verfestigung und der Abnahme vom Original mit der wäßrigen Lösung eines Desinfektionsmittels, das ausgewählt ist aus den Klassen Aldehyde, quartäre Ammoniumverbindungen, Biguanide, Aktivhalogenverbindungen und peroxidische Verbindungen, und die auch weitere Hilfsstoffe enthalten kann, in Kontakt gebracht werden, dadurch gekennzeichnet, daß diese Lösung 0,1 bis 15 Gewichtsprozent eines löslichen Alkohols oder eines Gemisches solcher Alkohole enthält. In der nicht vorveröffentlichten europäischen Patentanmeldung 308 786 werden zur Desinfektion von Abformmassen mikrobizide Phenole eingesetzt. Dies ist nicht Gegenstand der vorliegenden Erfindung.

Mit dem neuen Verfahren werden nicht nur eine schnelle und gründliche Abtötung aller relevanten Keime und Viren erreicht, sondern auch die Beeinträchtigungen der Maßhaltigkeit, der Oberflächengüte und der Ausgießbarkeit des Negativs so weit vermindert, daß sie in der Praxis nicht mehr stören. Dies gilt auch für Polyether und hydrophilisierte Silikone und selbst für die äußerst sensiblen polymeren Kohlenhydrate, insbesondere die Alginate, die in der Zahnmedizin als Abformmaterialien weite Verbreitung gefunden haben.

Das Verfahren wird meist in der Weise ausgeführt, daß die Negativform unmittelbar nach der Abnahme vom abzubildenden Organ oder, vorzugsweise, nach kurzem Abspülen mit Wasser in eine vorbereitete Lösung des Desinfektionsmittels soweit eingetaucht wird, daß alle möglicherweise kontaminierten Oberflächen von der Lösung benetzt werden. Die Lösung hat im allgemeinen Raumtemperatur, doch kann, wenn das Abformmaterial dies zuläßt, auch bei erhöhten Temperaturen desinfiziert werden. Die Kontaktzeiten richten sich nach dem gewünschten Desinfektionsgrad und liegen bei Raumtemperatur meistens im Bereich zwischen 1 und 20 Minuten, vorzugsweise zwischen 1 und 10 Minuten und insbesondere zwischen 1 und 5 Minuten. Die Form wird dann entnommen, meist noch einmal mit Wasser nachgespült und bis zur weiteren Verwendung in geeigneter Weise gelagert. Die Desinfektion muß allerdings nicht unbedingt im Anschluß an die Herstellung der Negativform durchgeführt werden, sondern kann auch zu einem späteren Zeitpunkt erfolgen, gegebenenfalls zusätzlich zu einem Desinfektionsgang unmittelbar nach Herstellung. Anstelle des bevorzugten Eintauchens kann die Form auch zur Desinfektion mit der Wirkstofflösung besprüht werden.

Die erfindungsgemäß einzusetzenden Alkohole sollen in der jeweiligen Einsatzkonzentration wasserlöslich sein. Geeignet sind beispielsweise Butandiol, Butanol, Triethylenglykol, Polyethylenglykole und gemischte Polyethylenpolypropylenglykole.

Vorzugsweise sind die Alkohole unbegrenzt mit Wasser mischbar. Bevorzugt werden deshalb Alkohole mit 1 bis 3 C-Atomen und Etheralkohole mit 3 bis 6 C-Atomen eingesetzt. Beispiele für Alkohole mit 1 bis 3 C-Atomen sind Ethanol, Isopropanol, n-Propanol, 1,2-Propylenglykol und Glycerin. Beispiele für Etheralkohole mit 3 bis 6 C-Atomen sind Diethylenglykol, Triethylenglykol und Butylglykol. Auch die in wäßriger Lösung überwiegend als Hydrate vorliegenden Aldehyde dieser Größe, beispielsweise Glyoxal, besitzen diese Wirkung, so daß sie die Alkohole zum Teil oder vollständig ersetzen können. Sie werden deshalb im Rahmen dieser Erfindung zu den Alkoholen gerechnet. Besonders bevorzugt werden Ethanol, n-Propanol und Glyoxal.

Durch den Zusatz der Alkohole zu den wäßrigen Desinfektionsmittellösungen wird überraschenderweise eine weit geringere Beeinträchtigung der Maßgenauigkeit und der Oberflächengüte bei der Desinfektion der Negativform erreicht als sie mit rein wäßrigen Lösungen oder überwiegend alkoholischen Lösungen der Desinfektionsmitteln zu erzielen ist. Die Konzentration an Alkohol liegt im allgemeinen zwischen etwa 0,1 und 15 Gewichtsprozent, bezogen auf das Gewicht der fertigen Desinfektionslösung, vorzugsweise zwischen 1 und 10 Gew.-%. Die am besten geeignete Konzentration hängt von der Art des Alkohols ab und liegt beispielsweise bei Isopropanol bei etwa 1 Gew.-%, bei n-Propanol und Glycerin bei etwa 2 bis 5 Gew.-%, bei 1,2 Propylenglykol bei etwa 1 bis 3 Gew.-% und bei Ethanol bei etwa 2 bis 6 Gew.-%.

Obwohl auch ein Teil der erfindungsgemäß einzusetzenden Alkohole eine gewisse - im Falle von Glyoxal oder Formaldehyd sogar starke - antimikrobielle Wirkung aufweisen, enthalten die Desinfektionslösungen zusätzlich übliche Desinfektionswirkstoffe. Die Auswahl der Wirkstoffe und die Wahl der Konzentration wird in erster Linie von dem zu bekämpfenden Keimspektrum aber auch von der beabsichtigten Desinfektionszeit bestimmt. Prinzipiell eignen sich als breit wirksame Desinfektionswirkstoffe beispielsweise Aldehyde, Phenole, quartäre Ammoniumverbindungen, Biguanide, Aktivhologenverbindungen und peroxidische Verbindungen, allein oder in geeigneter Kombination. Beispiele für geeignete Wirkstoffe aus diesen Klassen sind Glutaraldehyd, o-Phenylphenol, p-Chlor-m-kresol, Didecyldimethylammoniumchlorid, Benzyldimethylalkylammoniumchlorid, Oligohexamethylenbiguanid-Hydrochlorid, Wasserstoffperoxid und Peressigsäure. Gegebenenfalls kann es auch zweckmäßig sein, ein oder mehrere Wirkstoffe mit engem Keimspektrum, beispielsweise Fungizide, wie etwa Undecylensäurederivate, zuzusetzen. Besonders bevorzugte Wirkstoffe für das erfindungsgemäße Verfahren sind Aldehyde, insbesondere Succindialdehyd und Glutardialdehyd sowie peroxidische Verbindungen.

Für die meisten Fälle reichen Konzentrationen von 0,1 bis 10 Gew.-%, vorzugsweise 0,5 bis 7 Gew.-% an Desinfektionswirkstoff aus, doch ist es in besonderen Fällen durchaus möglich, auch außerhalb dieser Grenzen zu arbeiten. Enthalten die Desinfektionslösungen sowohl Glutardialdehyd als auch Glyoxal - eine bevorzugte Ausführungsform der Erfindung -, so werden diese Aldehyde vorzugsweise im Gewichtsverhältnis von 2 : 1 bis 1 : 10, insbesondere 2 : 1 bis 1 : 5 verwendet. Die Desinfektionslösung enthält die beiden Aldehyde vorzugsweise in einer Menge von 0,5 bis 7 Gew.-%, insbesondere 1 bis 5 Gew.-%, gerechnet als Summe beider Aldehyde. Daneben können bis zu 3 Gew.-%, vorzugsweise 0,01 bis 2 Gew.-%, an anderen Desinfektionswirkstoffen enthalten sein, doch kann in vielen Fällen auf den Zusatz weiterer Desinfektionswirkstoffe, auch verzichtet werden.

Zusätzlich zu den genannten Wirkstoffen können die Desinfektionsmittellösungen weitere übliche Hilfssubstanzen enthalten. Hier sind in erster Linie Tenside zu nennen, die die Benetzung der Negativform erleichtern sollen. Verwendet werden in erster Linie nichtionische und, vorzugsweise, anionische Tenside, doch können bei ausreichender Verträglichkeit mit den zu desinfizierenden Materialien auch andere Tensidtypen eingesetzt werden. (Nichtionische Tenside, d. h. im allgemeinen Additionsverbindungen aus Ethylenoxid und langkettigen Alkoholen oder Phenolen, zählen im Rahmen dieser Erfindung nicht zu den Alkoholen.) Daneben können pH-regulierende Stoffe und Puffersubstanzen enthalten sein, um den bevorzugten pH-Wert der Lösung von 2 bis 11, insbesondere 3 bis 9, sicher einzustellen. Als weitere Hilfsstoffe sind sequestrierend wirkende Substanzen, Duftstoffe, Farbstoffe, Schauminhibitoren und Hydrotrope zu nennen sowie Mittel, die die Abformbarkeit des Negativs mit Gips erleichtern, wie etwa anorganische Fluoride, beispielsweise Na₂TiF₆. Die Menge an Hilfsstoffen in der Desinfektionslösung liegt vorzugsweise zwischen 0,01 und 10 Gew.-% und insbesondere zwischen 0,1 und 5 Gew.-%. Die Menge an Tensiden macht vorzugsweise 0 bis etwa 1 Gew.-%, insbesondere 0 bis 0,5 Gew.-% und besonders bevorzugt 0,01 bis 0,3 Gew.-% der Lösung aus.

Die Desinfektionsmittellösungen können unmittelbar vor Anwendung des Verfahrens aus den Einzelkomponenten hergestellt werden, doch ist es bequemer und sicherer, bereits vorgefertigte Lösungen anzuwenden oder entsprechende Konzentrate nur zu verdünnen.

Eine für das erfindungsgemäße Verfahren besonders geeignete Desinfektionslösung hat folgende Zusammensetzung:
0,5 bis 5 Gew.-% Glyoxal,
0 bis 10 Gew.-% Alkohol aus der Gruppe Ethanol, n-Propanol und deren Mischungen,
0,5 bis 10 Gew.-% Aldehyd aus der Gruppe Succindialdehyd, Glutardialdehyd und deren Mischungen,
0 bis 3 Gew.-% eines oder mehrerer weiterer Desinfektionswirkstoffe,
0 bis 10 Gew.-% eines oder mehrerer Hilfsstoffe aus der Gruppe Tenside, pH-regulierende Substanzen, Sequestriermittel, Duftstoffe, Farbstoffe, Schauminhibitoren, Hydrotrope und anorganische Fluoride und
Rest zu 100 Gew.-% Wasser

Bevorzugtes Einsatzgebiet des erfindungsgemäßen Verfahrens ist die Desinfektion von Abdruckmassen auf dem Gebiet der Zahn- und Kiefermedizin. Das Verfahren ist aber keineswegs auf dieses Gebiet beschränkt, sondern nicht zuletzt wegen der außergewöhnlichen Materialverträglichkeit sehr breit anwendbar. In keinem Falle wurde beobachtet, daß der Zusatz der Alkohole die Brauchbarkeit der Mittel auch für andere Desinfektionsmaßnahmen eingeschränkt hätte.

### BEISPIELE

### 1. Desinfektionsmittellösungen

Die für die folgenden Prüfungen verwendeten wäßrigen Lösungen hatten folgende Zusammensetzung (in Gewichtsprozent, Rest Wasser):

| | A | B | C | D |
|---|---|---|---|---|
| Glyoxal | 0,88 | 1,76 | 0,88 | 0,88 |
| Glutaraldehyd | 0,45 | 0,90 | 0,45 | 0,45 |
| Ethanol | 5,5 | 5,5 | - | - |
| n-Propanol | - | - | 3,5 | - |
| Glycerin | - | - | - | 4,0 |
| Alkylbenzolsulfonat | 0,40 | 0,80 | 0,40 | 0,40 |
| Nichtionisches Tensid (EO-Addukt) | 0,20 | 0,40 | 0,20 | 0,20 |
| Sonstige Hilfsstoffe (Sequestriermitttel, Hydrotrop, Farbstoff) | 0,21 | 0,42 | 0,21 | 0,21 |

### 2. Materialverträglichkeit

Diese Prüfung wurde an Abformmassen auf Alginatbasis vorgenommen, die von allen heute in der zahnärztlichen Praxis verwendeten Abdruckmaterialien am empfindlichsten auf Fehlbehandlung reagieren. Als Testmaterial wurde das Produkt Palgat der Firma Espe, Seefeld, DE, verwendet. Der Test wurde wie folgt durchgeführt:

20 g des Alginats wurden mit 40 ml destilliertem Wasser gründlich vermischt und in die Probeform eingegossen ("apparatus for detail reproduction" nach ADA-Spezifikation Nr. 19 (American Dental Association), J. Am. Dent. Assoc., Vol. 84, April 1977, Seite 733 ff.) Überschüssiges Alginat wurde mit einer glatten Glasplatte verdrängt. Nach 15 Minuten Aushärtezeit bei Raumtemperatur (23° +/- 1° C) im Hygrophor (100 % rel. Luftfeuchtigkeit) wurde die Abformung entnommen und sofort in das Desinfektionsbad, enthaltend die Lösungen A, B, C und D eingegeben. Nach den in der Tabelle angegebenen Verweilzeiten im Desinfektionsbad (Temperatur 23° +/- 1° C) wurde die Abformung unter einem schwachen Strom Leitungswasser für 10 Sekunden gespült. Im Anschluß wurde die Abformung mit Gips (Moldano, Fa. Bayer, Leverkusen, DE) ausgegossen. Zur vollständigen Aushärtung des Gipsmodelles wurden Modell und Negativform 30 Minuten in einem Hygrophor (23° +/- 1° C, 100 % rel. Luftfeuchtigkeit) aufbewahrt. Die Alginatform wurde anschließend vorsichtig vom Gips getrennt und das Gipsmodell unter dem Mikroskop auf Dimensionsänderungen vermessen und auf Oberflächenbeschaffenheit untersucht.

Die Ergebnisse sind in der folgenden Tabelle 1 für verschiedene Einwirkungszeiten der Desinfektionslösung angegeben. Die Zahlenwerte bedeuten Prozent bei der Dimensionsänderung bzw. Bewertungszahl 1 - 3 (keine signifikanten Einflüsse; geringe Einflüsse - ohne Auswirkung auf die Präzision der Arbeit; starke, nicht tolerierbare Einflüsse) bei der Oberflächengüte des Gipsabgusses.

**TABELLE I**

| Lösung | Oberflächengüte | | | | | | Dimensionsänderung (n. Minuten) | | |
|---|---|---|---|---|---|---|---|---|---|
| | Alginat(n.Minuten) | | | Gips(n.Minuten) | | | | | |
| | 5 | 10 | 20 | 5 | 10 | 20 | 5 | 10 | 20 |
| A | 1 | 1 | 1 | 1-2 | 2 | 2 | -0,33 | -0,15 | -0,13 |
| B | 1 | 1 | 1 | 2 | 2 | 2 | -0,02 | -0,54 | -0,52 |
| C | 1 | 1 | 1 | 2 | 2 | 2 | +0,03 | -0,06 | -0,28 |
| D | 1 | 1 | n.b. | 1-2 | 1-2 | 3 | +0,28 | +0,06 | n.b. |

Aus den Werten wird deutlich, daß selbst nach Einwirkungszeiten von 10 bis 20 Minuten weder die Oberflächengüte noch die Maßgenauigkeit Veränderungen erfahren, die die Güte der prothetischen Arbeit beeinträchtigen könnten.

### 3. Desinfektionswirkung

Zur Prüfung der Desinfektionswirkung wurde Alginatabformmasse aus Wasser und dem Produkt Palgat (Fa. Espe, Seefeld, DE) nach Herstellerangaben im Gummibecher angerührt und durch Ausstreichen auf eine Tüpfelplatte aus Prozellan zu linsenförmigen Prüfkörpern von ca. 22 mm Durchmesser und 5 mm Dicke verarbeitet (Härtezeit 5 Minuten). Die Kontamination erfolgte anschließend durch Einlegen dieser Körper in eine Keimsuspension, die mehr als 10⁸ keimbildende Einheiten von Staphylococcus aureus pro Milliliter enthielt.

Nach der Kontaminination wurden die Keimträger in offenen Petrischalen in einem Exsiccator über Wasser 30 Minuten gelagert, dann zur Desinfektion für eine gewisse Zeit (5 oder 10 Minuten) in die Desinfektionslösung eingelegt, wobei auf vollständige Benetzung geachtet wurde. Zur Auszählung der noch lebensfähigen Keime wurden die Keimträger anschließend einzeln zusammen mit 10 g Glasperlen 2 Minuten in 20 ml einer Inaktivierungslösung geschüttelt und von dieser Lösung dann in üblicher Weise durch Überimpfen auf Agar und Bebrüten die Keimzahlen bestimmt.

Die Ergebnisse sind in der folgenden Tabelle II aufgeführt, wobei in logarithmischer Form die Faktoren der Keimzahlreduktion gegenüber einer Behandlung mit Wasser allein (als Standard) angegeben sind. Die einzelnen Zahlen sind Mittelwerte aus je 6 Einzelbestimmungen.

**TABELLE II**

| Lösung nach Beispiel | Reduktionsfaktor (log) nach | |
|---|---|---|
| | 5 Minuten | 10 Minuten |
| 1A | 5,1 | 5,2 |
| 1B | 5,5 | 5,5 |
| 1C | 4,9 | 5,3 |
| 1D | 5,5 | 5,1 |

Aus den Ergebnissen ist zu erkennen, daß in allen Fällen eine sichere Desinfektion der Formkörper erreicht wurde.

## Patentansprüche

1. Verfahren zur Desinfektion medizinischer Abformmassen, bei dem die Abformmassen nach der Verfestigung und der Abnahme vom Original mit der wäßrigen Lösung eines Desinfektionsmittels, das ausgewählt ist aus den Klassen Aldehyde, quartäre Ammoniumverbindungen, Biguanide, Aktivhalogenverbindungen und peroxidische Verbindungen, und die auch weitere Hilfsstoffe enthalten kann, in Kontakt gebracht werden, dadurch gekennzeichnet, daß diese Lösung 0,1 bis 15 Gewichtsprozent eines löslichen Alkohols oder eines Gemisches solcher Alkohole enthält.

2. Verfahren nach Anspruch 1, bei dem die Lösung einen Alkohol mit 1 bis 3 C-Atomen oder einen Etheralkohol mit 3 bis 6 C-Atomen oder ein Gemisch solcher Alkohole enthält.

3. Verfahren nach Anspruch 2, bei dem der Alkohol aus der Gruppe Ethanol, n-Propanol und Glyoxal ausgewählt ist.

4. Verfahren nach einem der Ansprüche 1 bis 3, bei dem 1 bis 10 Gew.-% an Alkohol eingesetzt werden.

5. Verfahren nach einem der Ansprüche 1 bis 4, bei dem die Lösung 0,1 bis 10 Gew.-%, vorzugsweise 0,5 bis 7 Gew.-%, an Desinfektionswirkstoff enthält.

6. Verfahren nach Anspruch 5, bei dem mindestens ein Desinfektionswirkstoff aus der Gruppe Succindialdehyd, Glutardialdehyd und peroxidische Verbindungen verwendet wird.

7. Verfahren nach einem der Ansprüche 1 bis 6, bei dem die Desinfektionslösung eine Kombination von Glutardialdehyd und Glyoxal im Gewichtsverhältnis von 2 : 1 bis 1 : 10, vorzugsweise von 2 : 1 bis 1 : 5, enthält.

8. Verfahren nach einem der Ansprüche 1 bis 7 zur Desinfektion von Alginat-Abformmassen.

## Claims

1. A process for the disinfection of medical moulding materials in which, after hardening and removal from the original, the moulding materials are contacted with an agueous solution of a disinfectant selected from the classes of aldehydes, quaternary ammonium compounds, biguanides, active halogen compounds and peroxide compounds, which may also contain other auxiliaries, characterized in that this solution contains from 0.1 to 15% by weight of a soluble alcohol or a mixture of soluble alcohols.

2. A process as claimed in claim 1 in which the solution contains a C₁₋₃ alcohol or a C₃₋₆ ether alcohol or a mixture of such alcohols.

3. A process as claimed in claim 2 in which the alcohol is selected from the group consisting of ethanol, n-propanol and glyoxal.

4. A process as claimed in any of claims 1 to 3 in which 1 to 10% by weight of alcohol are used.

5. A process as claimed in any of claims 1 to 4 in which the solution contains from 0.1 to 10% by weight and preferably from 0.5 to 7% by weight disinfecting agent.

6. A process as claimed in claim 5 in which at least one disinfecting agent from the group consisting of succindialdehyde, glutardialdehyde and peroxide compounds is used.

7. A process as claimed in any of claims 1 to 6 in which the disinfection solution contains a combination of glutardialdehyde and glyoxal in a ratio by weight of from 2:1 to 1:10 and preferably from 2:1 to 1:5.

8. A process as claimed in any of claims 1 to 7 for the disinfection of alginate moulding materials.

## Revendications

1. Procédé pour la désinfection d'empreintes médicales, dans lequel les empreintes sont mises en contact, après la solidification et l'enlèvement de l'original, avec la solution aqueuse d'un désinfectant qui est choisi dans les catégories des aldéhydes, des composés d'ammonium quaternaire, des biguanides, des composés renfermant des halogènes actifs et des peroxydes, et qui peut également contenir d'autres adjuvants, caractérisé en ce que cette solution contient de 0,1 à 15 % en poids d'un alcool soluble ou d'un mélange de tels alcools.

2. Procédé selon la revendication 1, dans lequel la solution contient un alcool ayant de 1 à 3 atomes de carbone ou un éther-alcool ayant de 3 à 6 atomes de carbone, ou un mélange de tels alcools.

3. Procédé selon la revendication 2, dans lequel l'alcool est choisi parmi l'éthanol, le n-propanol et le glyoxal.

4. Procédé selon l'une quelconque des revendications 1 à 3, dans lequel on utilise de 1 à 10 % en poids d'alcool.

5. Procédé selon l'une quelconque des revendications 1 à 4, dans lequel la solution contient de 0,1 à 10 % en poids, de préférence de 0,5 à 7 % en poids, de substance active désinfectante.

6. Procédé selon la revendication 5, dans lequel on utilise au moins une substance active désinfectante choisie parmi le dialdéhyde succinique, le glutardialdéhyde et les peroxydes.

7. Procédé selon l'une quelconque des revendications 1 à 6, dans lequel la solution de désinfection contient une association de glutardialdéhyde et de glyoxal en un rapport pondéral allant de 2:1 à 1:10, de préférence de 2:1 à 1:5.

8. Procédé selon l'une quelconque des revendications 1 à 7, pour la désinfection d'empreintes à base d'alginate.
